# EUROPEAN PATENT SPECIFICATION

(11) **EP 0 669 105 B1**
(45) Date of publication and mention of the grant of the patent: **02.08.2000**
(21) Application number: 95301168.1
(22) Date of filing: 22.02.1995
(51) Int. Cl.: A61B 17/32

(54) **Endoscopic resection instrument**
Endoskopisches Resezierinstrument
Appareil de résection endoscopique

(30) Priority: 23.02.1994 US 200662
(43) Date of publication of application: 30.08.1995
(73) Proprietor: SMITH & NEPHEW, INC., Memphis, Tennessee 38116 (US)
(72) Inventor: Smith, Graham, Plaistow, New Hampshire 03865 (US); Cesarini, Peter M., Londonderry, New Hampshire 03053 (US)
(74) Representative: Harrison Goddard Foote

(56) References cited:
- EP-A- 0 445 918
- EP-A- 0 481 760
- EP-A- 0 609 084
- EP-A- 0 613 661
- DE-A- 3 828 478

## Description

This invention relates to surgical instruments, and in particular to powered arthroscopic surgical instruments.

Powered arthroscopic surgical instruments typically include a rigid stationary outer tube within which a rigid inner tube is rotated by a motor. A cutting implement, such as a blade or abrading burr, is disposed on the distal end of the inner tube. Tissue or bone is exposed to the cutting implement through an opening in the distal end of the outer tube, and tissue or bone fragments cut by the rotating blade are drawn through the interior of the inner tube along with irrigating fluid by the use of suction applied at the proximal end of the instrument. Examples of such surgical instruments are described in US Patent Nos. 4,203,444; 4,274,414; 4,834,729; and 4,842,578, all of which are assigned to the present applicant.

Some arthroscopic surgical instruments are linear, that is, straight between their proximal and distal ends. Others are curved to facilitate positioning the cutting implement against tissue to be cut without requiring that the instrument be removed from the body and reinserted through an additional puncture. In a curved instrument, a region of the inner tube is flexible to enable the inner tube to accept the curvature imposed by the outer tube while transmitting the torque applied by the motor to the blade. In both cases, the user changes the orientation of the cutting implement by rotating the instrument.

The present invention provides a surgical instrument comprising
a rigid member having a bend region that angularly offsets a distal region of said rigid member from said proximal region;
a surgical device extending coaxially within said rigid member, said surgical device carrying a surgical tool distally of said bend region and having a relatively flexible region at least in said bend region to transmit a force applied at a proximal end of said surgical device through said bend region to operate said surgical tool;
wherein the surgical device comprises a second member coaxially disposed within a first member, the second member being movable with respect to the first member in response to said force,
characterised in that a proximal region of the rigid member is mounted to a first section of a base;
the first member is mounted to a second section of the base; and
said first section is rotatably mounted with respect to said second section to change in the operative state of the instrument a relative rotational orientation between said surgical tool and said bend region.

The preamble of the foregoing statement is indicative of the matter disclosed in German published specification (Offenlegegungsschrift) No 38 28 478 A1.

Among other advantages, the invention allows the user to change the relative rotational orientation between the surgical tool and the bend region. Accordingly, without turning the instrument and without changing the direction of offset of the bend region, the used can rotationally vary the direction in which cutting is performed. Alternatively, the user can, without turning the instrument and without changing the direction in which cutting is performed, change the direction of offset of the bend region. Thus, with only minimal manipulation of the instrument, the surgical tool can be positioned to cut tissue at a variety of different locations in a given surgical site.

Eliminating the need to turn the entire instrument is particularly useful with a curved instrument, because the distal region of the instrument is on an axis different from that of the remainder of the instrument. As a result, if the relative rotational orientation between the surgical tool and the bend region could not be changed, the entire instrument would have to be pivoted or otherwise swung about the axis of the distal region to rotate the surgical tool. Because it allows the instrument to remain in a fixed position while the rotational orientation of the surgical tool is changed with respect to the bend region, the present invention thereby facilitates the surgical procedure and reduces patient trauma and the risk of surgical side effects.

The surgical device comprises a second member (214) coaxially disposed within a first member (216), the first member being mounted to said second section of the base and the second member being movable with respect to the first member in response to said force.

In our earlier European Patent Application No. 94300614.8, published under serial number EP 0609084, on 3rd August 1994 after the priority date claimed for the present application, to which the reader is directed, there is disclosed a surgical instrument having a degree of similarity to that of the present invention. In that case, however, although the surgical device comprises first and second coaxial members with the second member being coaxially disposed within the rigid member, the rigid member is coaxially disposed within the first member.

Preferred embodiments include the following features.

Preferably, the first member is hollow and adapted to receive suction at its proximal end, allowing body material cut by the surgical tool to be transported away from the surgical site while the instrument remains in situ.

Both the first member and the second member are relieved (e.g., with a series of axially spaced, circumferentially extending slots) in the relatively flexible region. Pliable sheaths (such as heat-shrink tubing) are disposed over the first and second members to cover the slots. The pliable sheaths reduce the axial compressibility of the first and second members, minimize vacuum, tissue, fluid, and other leakage between the first, second, and rigid members, and protect the flexible regions from excessive wear as the first and second members rotate. The pliable sheath disposed over the slots of the second member further prevents the slots of the second member from interfering with the slots of the first member.

The surgical tool is preferably comprised of openings at the distal regions of the first and second members, the openings being arranged such that an edge of the opening in the first member moves toward and closely past an edge of the opening in the second member to cut tissue entering through the openings when force (e.g., torque) is applied (e.g., by a motor) to the first member.

The first section of the base is rotatable (e.g., manually by the user) with respect to the second section to allow the relative rotational orientation between the surgical tool and the bend region to be changed over a range of at least 360°. A ratchet mechanism allows the first section of the base to be selectively rotated to a plurality of discrete positions with respect to the second section, thereby also allow the relative rotational orientation between the surgical tool and the bend region to be selectively changed to a corresponding plurality of discrete relative rotational orientations.

Preferably, to provide this ratchet mechanism the second section includes a plurality of flexible cantilevered fingers, each of which corresponds to a discrete position, and the first section includes a plurality of mating regions, each of which engages one finger. This mechanism thereby avoids accidental rotation of the surgical tool with respect to the bend region.

Other features and advantages of the invention will become apparent from the following detailed description, and from the claims.

Fig. 1 shows an embodiment of a surgical instrument according to the invention.

Fig. 2 is a partial cross-sectional view of portions of the instrument of Fig. 1, showing details of the tip and base.

Figs. 3-5 show inner, intermediate, and outer tubes, respectively, of the surgical instrument of Fig. 1.

Fig. 6 is a cross-section of the surgical instrument, taken along line 6-6 of Fig. 2.

Fig. 7 shows the surgical instrument of Fig. 1 in use.

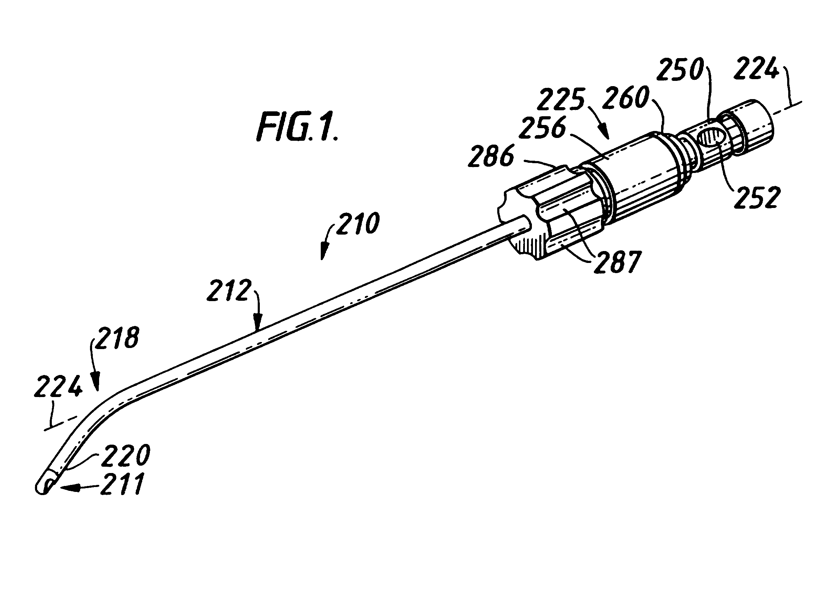

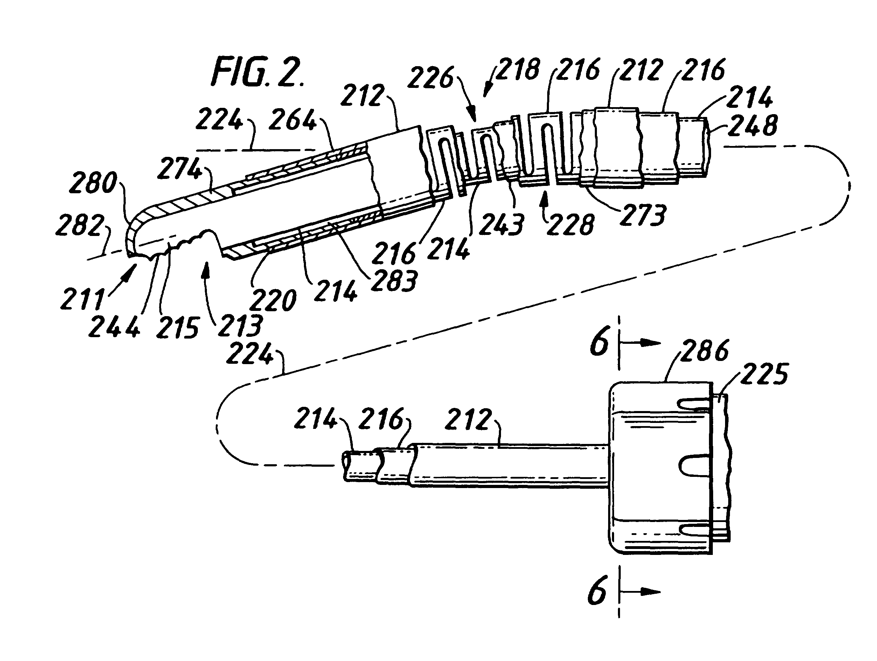

A surgical instrument in accordance with the invention is shown in Figs. 1 and 2. Surgical instrument 210 includes a rigid outer tube 212, within which a surgical device is coaxially disposed. The surgical device, which carries a surgical tool 211 at its distal end, is comprised of a rotating, partially flexible inner tube 214, coaxially disposed within partially flexible intermediate tube 216. Thus, whereas in the instrument previously described in EP-A-609 084 the rigid member 16 is sandwiched between the two tubes 12, 14 comprising the surgical device, the surgical device of surgical instrument 210 is disposed coaxially within rigid outer tube 212.

The distal end of intermediate tube 216 includes an opening 213, the edges of which are sharpened and serrated, through which a cutting implement 215 (formed by sharpened, serrated edges of a similar opening in the distal end of inner tube 214) of surgical tool 211 is periodically exposed as inner tube 214 rotates. Outer tube 212 is curved through a bend region 218 disposed slightly proximally of the distal end 220 of outer tube 212 to offset surgical tool 211 angularly from a generally straight axis 224 of surgical instrument 210.

Tubes 212, 214, and 216 are proximally supported by a base 225 constructed of, e.g., polycarbonate plastic. As discussed in detail below, outer tube 212 mounts to a knob 286 of base 225, and intermediate tube 216 mounts to a hub 256 of base 225.

Inner tube 214 includes a slotted, flexible region 226 disposed within bend region 218 to accept the curvature imposed by bend region 218 and transmit torque (and other forces) applied at base 225 through bend region 218 to rotate cutting implement 215 with sufficient force to sever tissue or other body material exposed through opening 213. Intermediate tube 216 also has a slotted, flexible region 228 disposed within bend region 218 to accept the curvature imposed by bend region 218. (For clarity, in Fig. 2 inner tube 214 is not shown behind slotted, flexible region 228 of intermediate tube 216.) Flexible region 228 allows the relative rotational orientation between opening 213 and bend region 218 to be changed, without interfering with the ability of inner tube 214 to rotate within intermediate tube 216. As described in detail below, this feature enables the user to maintain surgical instrument 210 in an essentially fixed position, while rotationally varying the direction in which cutting is performed without changing the direction of offset of the bend region 218. Alternatively, the user can, without changing the direction in which cutting is performed, change the direction of offset of bend region 218.

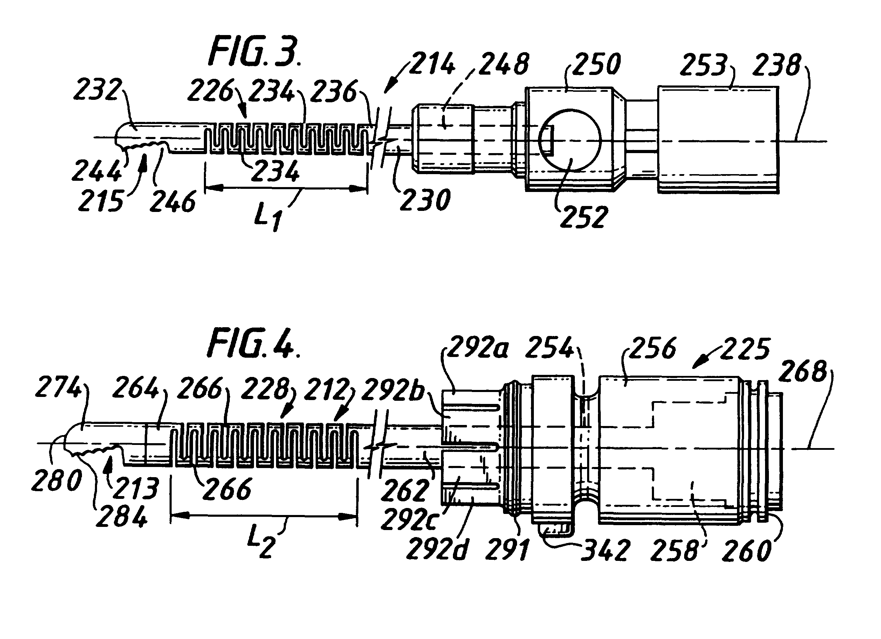

Referring also to Fig. 3, inner tube 214 is of generally the same construction as outer and inner tubes 12, 14 of the previously described instrument of EP-A-609 084. Rigid proximal and distal regions 230, 232 are connected by flexible region 226. Flexible region 226 is relieved with an axially extending series of circumferential slots 234 disposed in the walls 236 of tube 214, and is continuous with the adjacently disposed proximal and distal regions 230, 232. Each slot 234 is approximately 0.508 mm (0.020 inches) wide, and has a depth of about 3.429 mm (0.135 inches). The length L₁ of flexible region 226 should be sufficient, e.g. 17.78 mm (0.70 inches), that flexible region 226 spans the entire length of bend region 218.

A pliable sheath 243 (made from, e.g., heat-shrink tubing) slightly longer than L₁ is placed over inner tube 214 in bend region 218 to cover slots 234. (A portion of pliable sheath 243 in shown in Fig. 2.) Among other advantages, pliable sheath 43 helps prevent material from lodging within or passing through slots 234, and also helps prevent flexible region 226 from interfering with flexible region 228 as tubes 214, 216 rotate with respect to each other.

Distal region 232 of inner tube 214 supports cutting implement 215 (which is, for example, stainless steel and attached to tube 214 by welding or brazing). Cutting implement 215 is defined by serrated, sharpened edges 244 of a distal opening 246 in tube 214 and is sized to provide a close running fit with the interior surface of distal extension 274 of intermediate tube 216 (Figs. 2 and 4) for efficient cutting. Opening 246 is an extension of a central aperture 248 in inner tube that runs the entire length of tube 214 (see also Fig. 2). After cutting implement 215 is attached to inner tube 214, the outer surface of the entire assembly is ground to a uniform outer diameter. Optionally, the outer surface of inner tube 214 may then be plated with silver to provide an improved friction surface between inner tube 214 and intermediate tube 216.

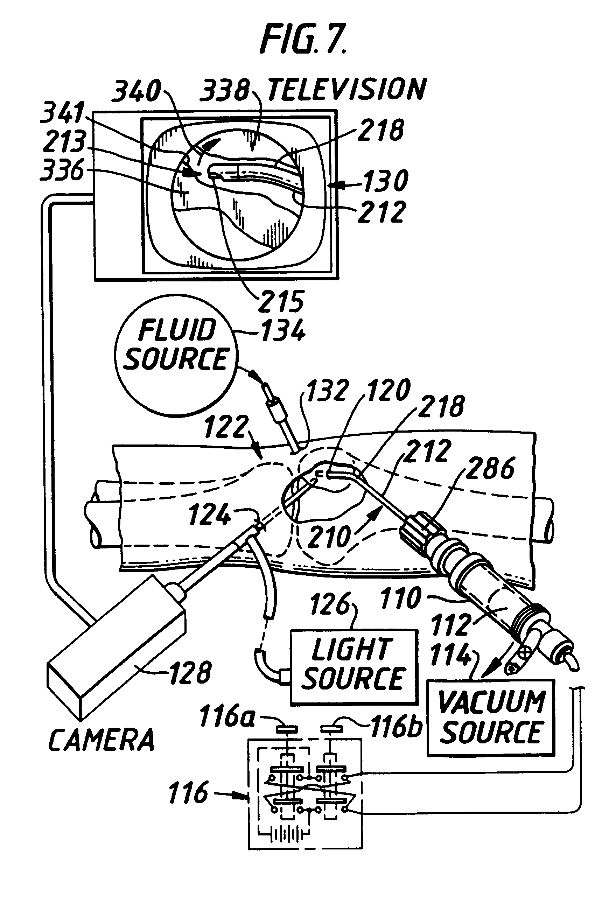

Proximal region 230 of inner tube 214 is mounted to a drive shaft 250 that rotates within base 225. Central aperture 248 terminates in a vacuum source opening 252 in drive shaft 250. The proximal end 253 of drive shaft 250 fits into a handpiece 310 (Fig. 7), which includes a motor 312 for rotating drive shaft 250 and inner tube 214 with respect to tubes 212 and 216. Opening 252 is coupled to a vacuum source 314 (Fig. 7) during operation to remove severed tissue and irrigating fluid from the surgical site via aperture 248 in a manner described in detail below.

As shown in Fig. 4, intermediate tube 216 is hollow along its entire length to provide a passage 254 that receives inner tube 214 (Fig. 2). The proximal end of intermediate tube 216 is rigidly mounted, for example by ultrasonic welding, to a hub 256 of base 225. A cavity 258 in hub 256 communicates with passage 254 and is configured to receive drive shaft 250. During assembly, inner tube 214 is inserted through hub 256 into intermediate tube 216 (before bend region 218 is formed in outer tube 212). A pliable fitting 260 retains drive shaft 250 within hub 256. Fitting 260 further provides a fluid-tight seal when base 225 is inserted into handpiece 310.

Intermediate tube 216 is essentially a larger version of inner tube 214 and includes rigid proximal and distal regions 262, 264 that are integrally connected by flexible region 228. Flexible region 228 is relieved with an axially extending series of circumferential slots 266. Each slot 266 is approximately 0.635 mm (0.025 inches) wide and has a depth of about 3.556 mm (0.140 inches). The length L₂ of flexible region 228 should be sufficient, e.g. 17.78 mm (0.70 inches), that flexible region 228 spans the entire length of bend region 218. A pliable sheath 273 (made from, e.g., heat-shrink tubing) slightly longer than L₂ is placed over intermediate tube 216 in bend region 218 to cover slots 266. (A portion of pliable sheath 273 in shown in Fig. 9.)

To provide opening 213, a hollow, closed-ended distal extension 274 having the same outer diameter as intermediate tube 216 is secured to intermediate tube 216 at distal end 264. Extension 274 is, for example, stainless steel, and is welded or brazed to intermediate tube 216, which can be made of, for example, stainless steel.

Opening 213 is disposed in a distal tip 280 of extension 274 and faces somewhat to the side of intermediate tube 216. That is, opening 213 does not along its entire length extend completely to the centerline 282 of extension 274 (Fig. 2). As a result, while surgical tool 211 will cut tissue that enters opening 213 from the distal end of instrument 210, the majority of the cutting action is to one side. Moreover, the inner surface of tip 280 provides distal support for the rotating inner tube 214. The edges 284 of opening 213 are sharpened and serrated to cooperate with sharp edges 244 of cutting implement 215.

As shown in Fig. 2, when instrument 210 is assembled, a slight gap 283 exists between the outer diameter of inner tube 214 and the inner diameter of intermediate tube 216. The gap 283 accommodates the thickness of pliable sheath 243 covering flexible region 226. Extension 274, however, has a reduced inner diameter with respect to the remainder of intermediate tube 216, such that the clearance between cutting implement 215 and the inner diameter of extension 274 is small, e.g. approximately 0.051 mm (0.002 inches). This arrangement maintains the close-running fit between edges 244, 284 while allowing inner tube 214 to rotate freely. The essentially identical inner diameters of extension 274 and inner tube 214 avoid cutting implement 215 scoring or seizing as it rotates.

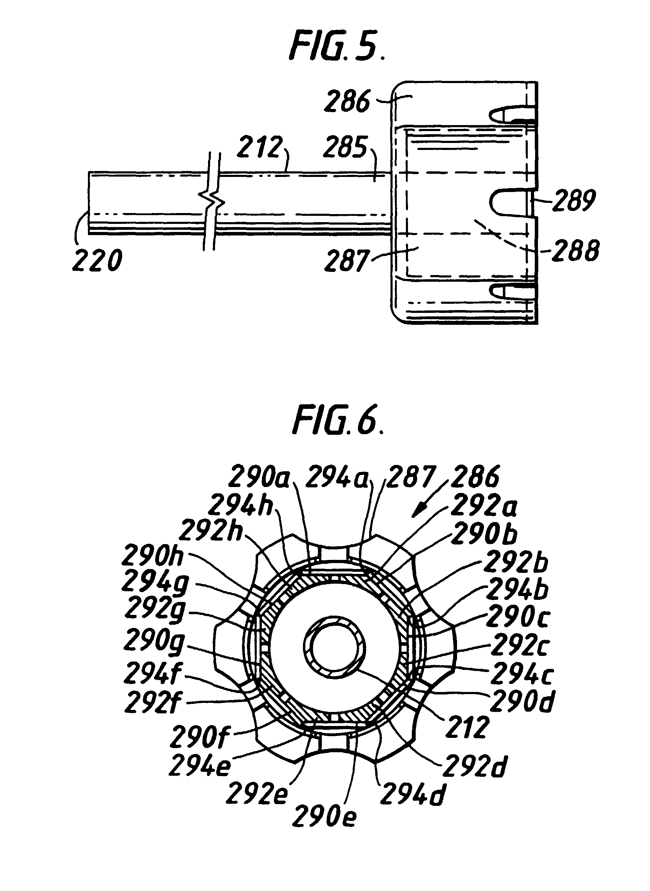

Fig. 5 shows the rigid member, outer tube 212 (before bend region 218 is formed), which is made from a rigid material such as metal (e.g., stainless steel). Proximal region 285 of outer tube 212 is rigidly secured, for example by ultrasonic welding, to a knob 286 at a sealed joint. A shoulder 289 on the inner surface of the proximal end of knob 286 engages a mating shoulder 291 on the outer surface of the distal end of hub 256 (Fig. 4), such that knob 286 rotatably mounts to hub 256 (see also Fig. 2). Thus, the relative rotational orientation between knob 286 and hub 256 can be changed, e.g., by grasping knob 286 and rotating hub 256, or by grasping hub 256 and rotating knob 286. The attachment mechanism connecting knob 286 and hub 256 is described in further detail below. Knob 286 is provided with a series of circumferentially spaced indentations 287 that facilitate the user's efforts manually to manipulate knob 286.

A central passage 288 extends through outer tube 212 and knob 286 to receive intermediate tube 216 and inner tube 214, which protrude through the open distal end 220 of outer tube 212. The inner diameter of outer tube 212 exceeds the outer diameter of intermediate tube 216 by a sufficient amount to accommodate pliable sheath 273 covering flexible region 228 (e.g., by approximately 0.005 inches, or 0.128 mm). This allows the user to change the relative rotational orientation between intermediate tube 216 and outer tube 212, but avoids excessive play or wobble between the intermediate and outer tubes 212, 216. After intermediate tube 216 is inserted into outer tube 212 and inner tube 214 is inserted into intermediate tube 216, outer tube 212 is curved to provide bend region 218 (Fig. 1).

When knob 286 is grasped firmly and hub 256 rotated, the rotational orientation of outer tube 212, and thus the direction of offset of bend region 218, remains fixed. Because proximal portion 262 of intermediate tube 216 is mounted to hub 256, rotating hub 256 also rotates intermediate tube 216 within outer tube 212. Intermediate tube 216 communicates this torque applied by the user at the base to extension 274 through flexible region 228 disposed in bend region 218. Thus, as hub 256 is rotated with respect to knob 286, the direction of offset of bend region 218 remains fixed, but opening 218 in extension 274 rotates with respect to bend region 218.

Alternatively, when hub 256 is grasped firmly and knob 288 is rotated, the directional orientation of opening 213 remains fixed (because hub 256 is also fixed), and the direction of offset of bend region 218 rotates (because knob 286 and outer tube 212 rotate). As the direction of offset of bend region 218 rotates, flexible region 228 allows the direction of offset of distal region 264 of intermediate tube 216 to rotate also.

Referring to Figs. 4 and 6 (Fig. 6 for clarity does not show tubes 214 and 216 in cross-section), the interior of knob 286 is octagonal in cross-section, its inner surface being composed of eight flat surfaces 290a-h of equal width. Cantilevered from the distal end of hub 256 are eight distally projecting flexible fingers 292a-h spaced by equal amounts (i.e., 45°) around the circumference of shoulder 291. Fingers 292a-h lie perpendicular to longitudinal axis 268 of intermediate tube 216. Each of fingers 292a-h is an irregular pentagon in cross-section, such that when knob 286 is assembled onto hub 256, the radial outermost point 294a-h of each finger 292a-h rests in an apex formed by the intersection of adjacent flat surfaces 290a-h.

Fingers 292a-h and flat surfaces 290a-h coact to allow the relative rotational orientation between knob 286 and hub 256 to be changed, in a ratchet-like fashion, in discrete, 45° steps. As the relative rotational orientation changes (i.e., as the knob 286 and hub 256 rotate with respect to one another), outermost points 294a-h move across flat surfaces 290a-h, initially forcing fingers 292a-h radially inward. When outermost points 294a-h move past the respective midpoints of the surfaces 290a-h, the elastic energy stored in the displaced flexible fingers 292a-h forces the fingers radially outward until relative rotational orientation between knob 286 and hub 256 has changed by 45°, and fingers 292a-h rest in the adjacent apex. Thus, fingers 290a-h positively urge outermost points 294a-h into each associated apex as it is encountered, thereby giving the surgeon kinesthetic feedback as to the amount by which opening 213 has been rotated, and helping to avoid accidental rotation of outer tube 212 with respect to hub 256.

Referring also to Fig. 7, in operation surgical instrument 210 is employed similarly to surgical instrument 10, as described in European Patent Application No.0609084.

As described, fluid introduced through a third puncture wound 132 from a fluid source 134 distends the body joint and irrigates the site, rendering tissue 136 (which is, e.g., synovial tissue) mobile so that it floats and can be displaced (similar to the movement of seaweed in water). Note that synovial tissue 336 is located beneath outer tube 212; thus, the rotational orientation between knob 286 and hub 256 is selected to produce the desired orientation between the bend region 218 and opening 213 (Figs. 2 and 6). The curvature provided by bend region 218 allows surgical instrument 210 to be easily positioned to place surgical tool 211 against tissue 336 (even if tissue 336 is located in a region of the joint that cannot easily be reached by a straight-shafted instrument) without manipulating instrument 210 unduly or requiring that additional punctures be made to gain access to tissue 336. This reduces patient discomfort, as well as the chances for infection and other deleterious consequences of the surgery.

The surgeon progressively cuts away synovial tissue 336 by moving surgical instrument 210 from side to side and in the axial direction using handpiece 310 (while viewing television screen 130). If during the procedure the surgeon wishes to cut tissue from another region of the synovial tissue, the present invention allows him to do so simply by changing the relative rotational orientation between surgical tool opening 213 and bend region 218.

For example, if the surgeon wishes to remove tissue from region 338 located above outer tube 212 (e.g., in the direction of arrow 340), he grasps knob 286 with the finger and thumb of one hand and turns handpiece 110, which in turn rotates hub 256. Handpiece 110 is provided with a distinct protuberance (not shown) that points in the same direction as tab 342 on hub 256 (Fig. 4) and opening 213 in surgical tool 211. Thus, the surgeon would continue to grasp knob 286 and rotate handpiece 110 until he tactually senses from the handpiece protuberance that opening 213 is properly oriented. The rotational force applied by the surgeon is transmitted through bend region 218 by flexible region 228, thereby causing distal extension 274 of intermediate tube 216 to rotate with respect to outer tube 212, changing the rotational orientation of opening 213 with respect to bend region 218 (in this case, by 180°).

Alternatively, if for example the surgeon wishes to change the direction of offset of bend region 218 without changing the direction of opening 213 (and thus the cutting direction of surgical tool 211), e.g. to remove tissue from region 341, he grasps handpiece 110 and rotates knob 286 with the finger and thumb of one hand. When the direction of offset of bend region 218 is as desired (in this case, when it had been rotated by 180°), the surgeon then moves the entire instrument axially until opening 213 was adjacent to region 341.

The surgeon can change the relative rotational orientation between bend region 218 and opening 213 at any time. For example, inner tube 214 can be driven by motor 112 or may be stationary while the surgeon rotates opening 213. Distal extension 274 rotates smoothly with respect to the stationary outer tube 212, while providing constant distal support (at tip 280) for rotating inner tube 214. The surgeon can return to cutting tissue 336 at any time simply by rotating handpiece 310 in either direction while holding knob 286 fixed (if the surgeon had been cutting from region 338), or by rotating knob 286 in either direction while holding handpiece 110 fixed (if the surgeon had been cutting from region 341).

Tissue fragments and other body material cut by surgical tool 211 are withdrawn from the surgical site along with irrigation fluid via central aperture 248 of inner tube 214 (Fig. 2) in response to suction applied by vacuum source 114. Flexible sheath 243 covering inner tube 214 ensures that tissue fragments, irrigation fluid, and other material do not pass through slots 234, thereby assisting in the transport of tissue fragments through the chamber and out of surgical instrument 210.

Although surgical instrument 210 is shown with opening 213 aligned in the same direction as tab 342 on hub 256 (Fig. 4), it is readily apparent that other alignments (e.g., in the opposite direction, to the right or left, or anywhere in between these directions) are possible. Indeed, a set of surgical instruments may be provided, each with a different opening 213 orientation, to give the user maximum flexibility in determining the optimum configuration for a given surgical procedure. In addition, outer tube 212 can be curved to any desired degree.

Surgical tools other than the cutting implement shown in the figures can be used. For example, the surgical tool can be configured to cut tissue exposed to distal tip 280 of intermediate tube 216. In this end cutter embodiment, coacting cutting edges are provided at the distal tips of both the inner and the intermediate tubes, and an axial bearing maintains the respective edges in close cutting relationship. Further, axial force can be used instead of, or in addition to, torque to operate the surgical tool. For example, the surgical tool could be a hinged punch or jaw assembly operated by an axial force applied at hub 256.

While the invention has been described in terms of surgical instruments for arthroscopy, the invention may also be used with other types of instruments, for example, instruments configured for other kinds of endoscopic procedures and for biopsy applications.

## Claims

1. A surgical instrument (210) comprising
a rigid member (212) having a bend region (218) that angularly offsets a distal region (220) of said rigid member from said proximal region;
a surgical device (214, 216) extending coaxially within said rigid member, said surgical device carrying a surgical tool (211), distally of said bend region and having a relatively flexible region (226, 228) at least in said bend region to transmit a force applied at a proximal end of said surgical device through said bend region to operate said surgical tool;
wherein the surgical device comprises a second member (214) coaxially disposed within a first member (216), the second member being movable with respect to the first member in response to said force,
characterised in that a proximal region of the rigid member is mounted to a first section (286) of a base (225);
the first member is mounted to a second section (256) of the base; and
said first section (286) is rotatably mounted with respect to said second section (256) to change in the operative state of the instrument a relative rotational orientation between said surgical tool and said bend region.

2. An instrument according to claim 1 wherein the first and second members are relieved in said relatively flexible region.

3. An instrument according to claim 2 wherein the first and second members are both relieved with a series of axially spaced, circumferentially extending slots (234, 266).

4. An instrument according to claim 3 further including a first pliable sheath (243) disposed to cover said slots of the second member, and a second pliable sheath (273) to cover said slots of the first member.

5. An instrument according to any preceding claim wherein the second member is hollow and is adapted to receive suction at its proximal end and to transport body material cut by the surgical tool away from a surgical site while the instrument remains in situ for further cutting.

6. A surgical tool according to any preceding claim wherein the surgical tool comprises a first opening (213) in a distal region of the second member and a second opening (246) in the distal region of the first member, an edge (284) of the first opening being arranged to move toward and closely past an edge (244) of the second opening in response to said force to cut tissue entering through the first and second openings.

7. An instrument according to claim 6 wherein said force is a torque applied by a motor (312) coupled to a proximal end of said second member, said relatively flexible region being configured to transmit said torque through said bend region to move said edge of the first member with respect to the second opening.

8. An instrument according to any preceding claim wherein the relative rotational orientation between said surgical tool and said bend region can be changed over a range of at least 360°.

9. An instrument according to any preceding claim wherein the relative rotational orientation between said surgical tool and said bend region can be changed manually by a user of the instrument.

10. An instrument according to any preceding claim wherein the relative rotational orientation between said first and second sections can be changed manually by a user of the instrument.

11. An instrument according to any preceding claim wherein the first section of the base can be selectively rotated to a plurality of discrete positions with respect to the second section of the base.

12. An instrument according to claim 11 wherein the second section of the base further includes a plurality of cantilevered fingers each of which corresponds to one of said discrete positions, the first section further including a plurality of mating regions, each said mating region engaging one of said fingers to maintain a selected discrete position.

## Patentansprüche

1. Chirurgisches Instrument (210) mit
einem starren Element (212), welches einen gebogenen Bereich (218) aufweist, welcher einen distalen Bereich (220) des starren Elementes winkelmäßig gegenüber dem proximalen Bereich versetzt,
einem chirurgischen Gerät (214, 216), welches sich koaxial innerhalb des starren Elementes erstreckt und ein chirurgisches Werkzeug (211) distal von dem gebogenen Bereich trägt und einen relativ flexiblen Bereich (226, 228) wenigstens in dem gebogenen Bereich aufweist, um eine Kraft, die an einem proximalen Ende des chirurgischen Gerätes aufgebracht wird, durch den gebogenen Bereich zu übertragen, um das chirurgische Werkzeug zu betätigen,
wobei das chirurgische Gerät ein zweites Element (214) aufweist, welches koaxial innerhalb eines ersten Elementes (216) angeordnet ist, und das zweite Element in bezug auf das erste Element in Abhängigkeit von dieser Kraft bewegbar ist,
**dadurch gekennzeichnet**, dass ein proximaler Bereich des starren Elementes an einem ersten Abschnitt (286) einer Halterung (225) angebracht ist,
das erste Element an einem zweiten Abschnitt (256) der Halterung angebracht ist, und
der erste Abschnitt (286) in bezug auf den zweiten Abschnitt (256) rotierbar angebracht ist, um im Betriebszustand des Instrumentes eine relative Rotations-Orientierung zwischen dem chirurgischen Werkzeug und dem gebogenen Bereich zu ändern.

2. Instrument nach Anspruch 1, bei welchem erstes Element und zweites Element im relativ flexiblen Bereich reduziert sind.

3. Instrument nach Anspruch 2, bei welchem erstes Element und zweites Element durch eine Serie von in axialen Abständen voneinander angeordneten, sich in Umfangsrichtung erstreckenden Schlitzen (234, 266) reduziert sind.

4. Instrument nach Anspruch 3, welches weiterhin eine erste biegsame Hülle (243), die angeordnet ist, um die Schlitze des zweiten Elementes abzudecken, und eine zweite biegsame Hülle (273) aufweist, um die Schlitze des ersten Elementes abzudekken.

5. Instrument nach einem der vorhergehenden Ansprüche, bei welchem das zweite Element hohl und so ausgebildet ist, daß es an seinem proximalen Ende Unterdruck aufnehmen und Körpermaterial, welches durch das chirurgische Werkzeug abgeschnitten worden ist, von der Operationsstelle wegtransportieren kann, während das Instrument für weitere Schneidarbeit an Ort und Stelle verbleibt.

6. Chirurgisches Werkzeug nach einem der vorhergehenden Ansprüche, bei welchem das chirurgische Werkzeug eine erste Öffnung (213) in einem distalen Bereich des zweiten Instrumentes und eine zweite Öffnung (246) im distalen Bereich des ersten Elementes aufweist, wobei eine Kante (284) der ersten Öffnung so angeordnet ist, dass sie sich in Richtung auf und dicht an einer Kante (244) der zweiten Öffnung in Abhängigkeit von der Kraft vorbeibewegt, um Gewebe, welches durch erste und zweite Öffnung eintritt, abzuschneiden.

7. Instrument nach Anspruch 6, bei welchem diese Kraft ein Drehmoment ist, welches durch einen Motor (312) aufgebracht wird, der mit einem proximalen Ende des zweiten Elementes verbunden ist, wobei der relativ flexible Bereich so konfiguriert ist, daß er das Drehmoment durch den gebogenen Bereich überträgt, um die Kante des ersten Elementes in bezug auf die zweite Öffnung zu bewegen.

8. Instrument nach einem der vorhergehenden Ansprüche, bei welcher die relative Rotations-Orientierung zwischen dem chirurgischen Werkzeug und dem gebogenen Bereich über einen Bereich von wenigstens 360° geändert werden kann.

9. Instrument nach einem der vorhergehenden Ansprüche, bei welchem die relative Rotations-Orientierung zwischen dem chirurgischen Werkzeug und dem gebogenen Bereich durch den Benutzer des Instrumentes manuell geändert werden kann.

10. Instrument nach einem der vorhergehenden Ansprüche, bei welchem die relative Rotations-Orientierung zwischen erstem und zweitem Abschnitt durch einen Benutzer des Instrumentes manuell geändert werden kann.

11. Instrument der vorhergehenden Ansprüche, bei welchem der erste Abschnitt der Halterung wahlweise in mehrere diskrete Positionen in bezug auf den zweiten Abschnitt der Halterung rotiert werden kann.

12. Instrument nach Anspruch 11, bei welchem der zweite Abschnitt der Halterung weiterhin mehrere auskragende Finger aufweist, von denen jeder einer der diskreten Positionen entspricht, und der erste Abschnitt weiterhin mehrere zugehörige Bereiche aufweist und jeder zugehörige Bereich mit einem der Finger in Eingriff kommt, um eine gewählte diskrete Position aufrechtzuerhalten.

## Revendications

1. Instrument chirurgical (210) comprenant :
un élément rigide (212) ayant une région coudée (218) qui décale angulairement une région distale (220) dudit élément rigide par rapport à une région proximale ;
un dispositif chirurgical (214,216) s'étendant coaxialement à l'intérieur dudit élément rigide, ledit dispositif chirurgical portant un outil chirurgical (211) distalement par rapport à ladite région coudée et comportant une région relativement flexible (226,228), au moins dans ladite région coudée, pour transmettre une force appliquée à une extrémité proximale dudit dispositif chirurgical, à travers ladite région coudée, afin d'actionner ledit outil chirurgical ;
dans lequel le dispositif chirurgical comprend un deuxième élément (214) coaxialement placé à l'intérieur d'un premier élément (216), le deuxième élément étant déplaçable par rapport au premier élément en réponse à ladite force ;
caractérisé en ce que :
une région proximale de l'élément rigide est montée sur une première partie (286) d'une base (225) ;
le premier élément est monté sur une deuxième partie (256) de la base ; et
ladite première partie (286) est montée de façon tournante par rapport à ladite deuxième partie (256) pour modifier, dans l'état de fonctionnement de l'instrument, une orientation en rotation relative entre ledit outil chirurgical et ladite région coudée.

2. Instrument selon la revendication 1, dans lequel les premier et deuxième éléments sont assouplis dans ladite région relativement flexible.

3. Instrument selon la revendication 2, dans lequel les premier et deuxième éléments sont tous deux assouplis par une série de fentes axialement espacées et s'étendant circonférentiellement (234,266).

4. Instrument selon la revendication 3, comprenant en outre une première gaine souple (243) disposée de manière à couvrir lesdites fentes du deuxième élément, et une deuxième gaine souple (273) disposée de manière à couvrir lesdites fentes du premier élément.

5. Instrument selon une quelconque des revendications précédentes, dans lequel le deuxième élément est creux et peut recevoir une aspiration à son extrémité proximale et transporter la matière du corps coupée par l'outil chirurgical afin de l'éloigner d'un site chirurgical tandis que l'instrument reste en place pour continuer la coupe.

6. Outil chirurgical selon une quelconque des revendications précédentes, dans lequel l'outil chirurgical comprend une première ouverture (213) dans une région distale du deuxième élément et une deuxième ouverture (246) dans la région distale du premier élément, un bord (284) de la première ouverture étant disposé de manière à se déplacer vers un bord (244) de la deuxième ouverture et à passer très près de ce bord en réponse à ladite force, afin de couper le tissu entrant à travers les première et deuxième ouvertures.

7. Instrument selon la revendication 6, dans lequel ladite force est un couple appliqué par un moteur (312) accouplé à une extrémité proximale dudit deuxième élément, ladite région relativement flexible étant configurée pour transmettre ledit couple à travers ladite région coudée afin de déplacer ledit bord du premier élément par rapport à la deuxième ouverture.

8. Instrument selon une quelconque des revendications précédentes, dans lequel l'orientation en rotation relative entre ledit outil chirurgical et ladite région coudée peut être modifiée dans une plage d'au moins 360 degrés.

9. Instrument selon une quelconque des revendications précédentes, dans lequel l'orientation en rotation relative entre ledit outil chirurgical et ladite région coudée peut être modifiée manuellement par un utilisateur de l'instrument.

10. Instrument selon une quelconque des revendications précédentes, dans lequel l'orientation en rotation relative entre lesdites première et deuxième parties peut être modifiée manuellement par un utilisateur de l'instrument.

11. Instrument selon une quelconque des revendications précédentes, dans lequel on peut faire tourner sélectivement la première partie de la base à une pluralité de positions distinctes par rapport à la deuxième partie de la base.

12. Instrument selon la revendication 11, dans lequel la deuxième partie de la base comprend en outre une pluralité de doigts en porte-à-faux dont chacun correspond à une desdites positions distinctes, la première partie comprenant en outre une pluralité de régions d'enclenchement, chaque dite région d'enclenchement venant en prise avec un desdits doigts pour maintenir une position distincte choisie.
